# EUROPÄISCHE PATENTSCHRIFT

(11) **EP 2 229 367 B1**
(45) Veröffentlichungstag und Bekanntmachung des Hinweises auf die Patenterteilung: **25.02.2015**
(21) Anmeldenummer: 08859229.0
(22) Anmeldetag: 08.12.2008
(51) Int. Cl.: C07D 233/58, C07D 233/60

(54) **VERFAHREN ZUR HERSTELLUNG VON DISUBSTITUIERTEN IMIDAZOLIUMSALZEN**
METHOD FOR THE PRODUCTION OF DISUBSTITUTED IMIDAZOLIUM SALTS
PROCÉDÉ DE PRODUCTION DE SELS D'IMIDAZOLIUM DISUBSTITUÉS

(30) Priorität: 12.12.2007 EP 07122982
(43) Veröffentlichungstag der Anmeldung: 22.09.2010
(73) Patentinhaber: BASF SE, 67056 Ludwigshafen (DE)
(72) Erfinder: SIEMER, Michael, 68159 Mannheim (DE); DEGEN, Georg, 64653 Lorsch (DE); GROLL, Peter, 67125 Dannstadt-Schauernheim (DE)
(86) Internationale Anmeldenummer: PCT/EP2008/067014
(87) Internationale Veröffentlichungsnummer: WO 2009/074535

(56) Entgegenhaltungen:
- WO-A-91/14678
- WO-A-02/094883
- WO-A-2006/027069
- WO-A-2007/076979
- WO-A-2008/151034
- WO-A-2009/027250
- VYGODSKII Y S; LOZINSKAYA E I; SHAPLOV A S; LYSSENKO K A; ANTIPIN M Y; URMAN Y G: "Implementation of ionic liquids as activating media for polycondensation processes" POLYMER, ELSEVIER SCIENCE PUBLISHERS B.V, GB, Bd. 45, Nr. 15, 12. Juli 2004 (2004-07-12), Seiten 5031-5045, XP004518193 ISSN: 0032-3861
- DEREK M WOLFE; PETER R SCHREINER: "Oxidative Desulfurization of Azole-2-thiones with Benzoyl Peroxide: Syntheses of Ionic Liquids and Other Azolium Salts" EUROPEAN JOURNAL OF INORGANIC CHEMISTRY, WILEY-VCH VERLAG, WEINHEIM, DE, Bd. 2007, Nr. 17, 1. Juni 2007 (2007-06-01), Seiten 2825-2838, XP008111195 ISSN: 1434-1948
- SERGEI V DZYUBA AND RICHARD A BARTSCH: "New room-temperature ionic liquids with C2-symmetrical imidazolium cations" CHEMICAL COMMUNICATIONS - CHEMCOM, ROYAL SOCIETY OF CHEMISTRY, GB, Nr. 16, 1. Januar 2001 (2001-01-01), Seiten 1466-1467, XP008111201 ISSN: 1359-7345

## Beschreibung

Die Erfindung betrifft ein Verfahren zur Herstellung von 1,3 disubstituierten Imidazoliumsalzen der Formel I 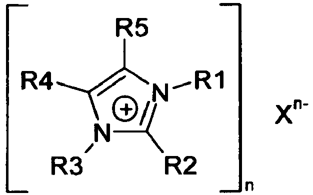 worin
R1 und R3 jeweils unabhängig voneinander für einen organischen Rest mit 1 bis 20 C-Atomen stehen,
R2, R4, und R5 unabhängig voneinander für ein H-Atom oder für einen organischen Rest mit 1 bis 20 C-Atomen stehen,
X für das Anion einer Wasserstoffsäure mit einem pKₛ-Wert von mindestens 2 (gemessen bei 25 °C, 1 bar in Wasser oder Dimethylsulfoxid) steht, und
n für 1, 2 oder 3 steht,
und welche eine Löslichkeit in Wasser beziehungsweise Mischbarkeit mit Wasser von mindestens 50 Gramm Imidazoliumsalz in 1 Liter Wasser (bei 1 bar, 21 °C) haben,
und das Verfahren dadurch gekennzeichnet ist, dass
   a) eine α -Dicarbonylverbindung, ein Aldehyd, ein Amin und die Wasserstoffsäure des Anions X⁻ miteinander umgesetzt werden und
   b) die Umsetzung in Wasser, einem mit Wasser mischbaren Lösemittel oder deren Gemische erfolgt und das Reaktionsgemisch weder während noch nach der Umsetzung Kohlenwasserstoffe enthält.

Imidazoliumsalze haben eine große anwendungstechnische Bedeutung als ionische Flüssigkeiten. Unter dem Begriff ionische Flüssigkeiten werden Salze mit einem Schmelzpunkt kleiner 200°C, insbesondere bei Raumtemperatur flüssige Salze verstanden.

Ionische Flüssigkeiten, insbesondere Imidazoliumsalze, eignen sich z. B. als Lösemittel in viele technische Anwendungen, z. B. auch für das Lösen von Zellulose.

Gewünscht sind daher möglichst einfache und kostengünstige Verfahren zur Herstellung derartiger Imidazoliumsalze in möglichst hoher Reinheit und Qualität. In WO 91/14678 wird ein einstufiges Verfahren zur Herstellung von Imidazoliumsalzen aus einer α -Dicarbonylverbindung, einem Aldehyd, einem Amin und einer Säure beschrieben. Wasser wird durch azeotrope Destillation mit Toluol als Schleppmittel entfernt. Das beschriebene Verfahren ist diskontinuierlich, mit einer azeotropen Destillation ist ein kontinuierliches Verfahren im allgemeinen nicht möglich.

Bei dieser Umsetzung fallen unerwünschte Nebenprodukte an, insbesondere das Ammoniumsalz der verwendeten Säure. Bevorzugte Säuren sind starke Säuren mit einem pKₛ Wert kleiner 4.Weiterhin erfolgt die Umsetzung in einem organischen Lösmittel. Das organische Lösmittel und das bei der Umsetzung entstandene Wasser müssen aufwendig durch azeotrope Destillation entfernt werden. Aufgrund sonstiger Nebenprodukte hat das erhaltene Reaktionsprodukt eine dunkle bis schwarze Färbung.

Ein entsprechendes Verfahren ist auch aus WO 02/94883 bekannt. Als Anionen werden hier hydrohobe, z. B. fluorierte Anionen verwendet, damit die Produkte separate Phase anfallen und leicht von der wässrigen Phase abgetrennt werden können. Auch das hier beschriebene Verfahren ist diskontinuierlich.

Die bisher bekannten einstufigen Verfahren zur Herstellung von Imidazolium-salzen erfüllen daher die obigen Anforderungen noch nicht im ausreichenden Maße.

Aufgabe der vorliegenden Erfindung ist daher ein Verfahren zur Herstellung von Imidazoliumsalzen, welches möglichst einfach durchzuführen und daher kostengünstig ist und welches Reaktionsprodukte in hoher Ausbeute und Qualität ergibt.

Demgemäß wurde das eingangs definierte Verfahren gefunden.

Zu den Imidazoliumsalzen

Es werden 1,3 disubstituierte Imidazoliumsalze der Formel I 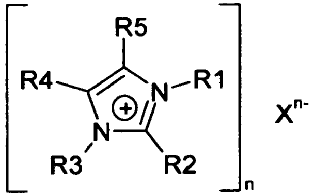 worin
R1 und R3 jeweils unabhängig voneinander für einen organischen Rest mit 1 bis 20 C-Atomen stehen
R2, R4, und R5 unabhängig voneinander für ein H-Atom oder für einen organischen Rest mit 1 bis 20 C-Atomen stehen
X für das Anion einer Wasserstoffsäure mit einem pKₛ -Wert von mindestens 2 (gemessen bei 25 °C, 1 bar in Wasser oder Dimethylsulfoxid) steht, und
n für 1, 2 oder 3 steht, hergestellt.
R1 und R3 stehen vorzugsweise unabhängig voneinander für einen organischen Rest mit 1 bis 10 C-Atomen. Der organische Rest kann auch noch weitere Heteroatome, insbesondere Sauerstoffatome, zum Beispiel Hydroxylgruppen, Ethergruppen, Estergruppen oder Carbonylgruppen enthalten.

Insbesondere stehen R1 und R3 für einen Kohlenwasserstoffrest, der außer Kohlenstoff und Wasserstoff allenfalls noch Hydroxylgruppen, Ethergruppen, Estergruppen oder Carbonylgruppen enthalten kann.

R1 und R3 stehen besonders bevorzugt unabhängig voneinander für einen Kohlenwasserstoffrest mit 1 bis 20 C- Atomen, insbesondere mit 1 bis 10 C-Atomen, der keine sonstigen Heteroatome, z. B. Sauerstoff oder Stickstoff, enthält. Der Kohlenwasserstoffrest kann aliphatisch (wobei auch ungesättigte aliphatische Gruppen eingeschlossen sind) oder aromatisch sein oder sowohl aromatische als auch aliphatische Gruppen enthalten. Vorzugsweise stehen R1 und R2 für einen aliphatischen Kohlenwasserstoffrest.

Als Kohlenwasserstoffreste genannt seien z.B. die Phenylgruppe, Benzylgruppe, eine durch eine oder mehrere C1 bis C4 Alkylgruppen substituierte Phenylgruppe oder Benzylgruppe, Alkylgruppen und Alkenylgruppen, insbesondere die Allylgruppe. Ganz besonders bevorzugt stehen R1 und R3 für eine C1 bis C10 Alkylgruppe. Als Alkylgruppe ist eine C1 bis C6 Alkylgruppe besonders bevorzugt, in einer besonderen Ausführungsform handelt es sich bei der Alkylgruppe um eine C1 bis C4 Alkylgruppe.

Ganz besonders bevorzugt stehen R1 und R3 unabhängig voneinander für eine Methyl-, Ethyl-, n-Propyl-, Isopropyl-, n-Butyl-, sec.Butyl oder tert.Butylgruppe, eine, wobei die Methyl-, Ethyl-, n-Propyl- und n-Butylgruppe besondere Bedeutung haben.

In einer bevorzugten Ausführungsform stehen R1 und R3 jeweils für den gleichen organischen Rest, bei den Imidazoliumsalzen der Formel I handelt es sich daher insbesondere um symmetrische, disubstituierte Imidazoliumsalze.
In einer ebenfalls bevorzugten Ausführungsform handelt es sich bei den Imidazoliumsalzen um Gemische von Imidazoliumsalzen der Formel I mit unterschiedlichen Resten R1 und R3. Derartige Gemische sind erhältlich durch Verwendung von unterschiedlichen Aminen, z. bei primären Aminen mit unterschiedlichen Alkylgruppen. Das erhaltenen Gemisch enthält dann sowohl Imidazoliumsalze, in denen R1 und r3 identisch sind als auch Imidazolumsalze, in denen R1 und R3 unterschiedliche Bedeutung haben.

In einer besonderen Ausführungsform stehen
R1 und R3 für eine Methylgruppe,
R1 und R3 für eine Ethylgruppe,
R1 und R3 für eine n-Propylgruppe,
R1 und R3 für eine n-Butylgruppe
R1 für eine Methylgruppe und R3 für eine Ethylgruppe
R1 für eine Methylgruppe und R3 für eine n Propylgruppe
R1 für eine Methylgruppe und R3 für eine n Butylgruppe
R1 für eine Methylgruppe und R3 für eine Allylgruppe
R1 für eine Ethylgruppe und R3 für eine Allylgruppe
R1 für eine Methylgruppe und R3 für eine Benzylgruppe
R1 für eine Ethylgruppe und R3 für eine Benzylgruppe

R2, R4, und R5 stehen unabhängig voneinander für ein H-Atom oder für einen organischen Rest mit 1 bis 20 C-Atomen stehen, wobei R4 und R5 auch zusammen einen aliphatischen oder aromatischen Ring ausbilden können. Der organische Rest kann neben Kohlenstoff und Wasserstoff auch Heteroatome wie Stickstoff oder Sauerstoff enthalten; vorzugsweise kann er Sauerstoff enthalten, insbesondere in Form von Hydroxylgruppen, Estergruppen, Ethergruppen oder Carbonylgruppen.

Insbesondere stehen R2, R4 und R5 unabhängig voneinander für ein H-Atom oder einen Kohlenwasserstoffrest, der außer Kohlenstoff und Wasserstoff allenfalls noch Hydroxylgruppen, Ethergruppen, Estergruppen oder Carbonylgruppen enthalten kann.

R2, R4 und R5 stehen bevorzugt unabhängig voneinander für ein Wasserstoffatom oder einen Kohlenwasserstoffrest mit 1 bis 20 C- Atomen, insbesondere mit 1 bis 10 C-Atomen, der keine sonstigen Heteroatome, z. B. Sauerstoff oder Stickstoff, enthält. Der Kohlenwasserstoffrest kann aliphatisch (wobei auch ungesättigte aliphatische Gruppen eingeschlossen sind) oder aromatisch sein oder sowohl aus aromatischen als auch aus aliphatischen Gruppen bestehen, wobei R4 und R5 auch auch einen aromatischen oder aliphatischen Kohlewasserstoffing ausbilden können, der gegebenenfalls durch weitere aromatische oder aliphatische Kohlenwasserstoffgruppen substituiert sein kann (die Anzahl der C-Atome des gegebenenfalls substituierten Kohlenwasserstoffrings einschließlich der Substituenten kann dabei vorzugsweise maximal 40, insbesondere maximal 20, besonders bevorzugt maximal 15 bzw. maximal 10 betragen).
Als Kohlenwasserstoffreste genannt seien z.B. die Phenylgruppe, eine Benzylgruppe, eine durch eine oder mehrere C1 bis C4 Alkylgruppen substituierte Phenylgruppe oder Benzylgruppe, Alkylgruppen, Alkenylgruppen und, im Falle, dass R4 und R5 einen Ring ausbilden, ein durch R4 und R5 ausgebildeter aromatischer 5 - oder 6 Ring, ein Cyclohexen - oder Cyclpenten, wobei diese Ringsysteme insbesondere durch eine oder mehrere C1 bis C10, insbesondere C1 bis C4 Alkylgruppen substituiert sein können.
Als Kohlenwasserstoffrest sind aliphatische Kohlenwasserstoffreste bevorzugt.

Besonders bevorzugt stehen R2, R4 und R5 unabhängig voneinander für ein H-Atom, eine C1 bis C8 Alkylgruppe oder eine C1-C8 Alkenylgruppe, z. B. eine Allygruppe,

Ganz besonders bevorzugt stehen R2, R4 und R5 unabhängig voneinander für ein H-Atom, eine Methyl-, Ethyl-, n Propyl-, Isopropyl-, n Butyl-, sec.Butyl oder tert.Butylgruppe, wobei die Methyl-, Ethyl-, n Propyl- und n Butylgruppe besondere Bedeutung haben.

In einer besonderen Ausführungsform steht R2 unabhängig von den anderen Resten R 4 und R5 und den übrigen Resten R1 und R3 für ein H-Atom. Imidazoliumsalze der Formel I, in denen R2 für ein H Atom steht, sind im Rahmen der vorliegenden Erfindung besonders vorteilhaft, sie haben eine gute Löslichkeit in den Epoxyverbindungen und eine hohe Wirksamkeit als latenter Katalysator. In einer besonderen Ausführungsform steht R2 für ein H-Atom, wenn es sich bei dem Anion um ein Acetat handelt.

In einer besonderen Ausführungsform stehen
R2, R4 und R5 für ein H-Atom,
R2 für ein H-Atom oder eine C1 bis C4 Alkylgruppe und R4, R5 für ein H-Atom oder eine C1 bis C4 Alkylgruppe

Als Einzelfälle für die Kationen der Verbindungen der Formel I seien genannt:
1-Butyl-3-methyl-imidazolium (R1= Butyl, R3 = Methyl)
1-Butyl-3-ethyl-imidazolium (R1=Butyl, R3=Ethyl)
1,3-Di-methyl-imidazolium (R1= Methyl, R3 =Methyl)
1-Ethyl-3-methyl-imidazolium (R1=Ethyl, R3 =Methyl)
1-Ethyl-2,3 dimethyl- imidazolium (R1 =Ethyl, R2=Methyl, R3=Methyl)

In Formel I steht n für 1, 2 oder 3; das Anion hat entsprechend ein, zwei oder drei negative Ladungen und entsprechend liegen im Salz ein, zwei oder drei Imidazoliumkationen vor.

Bevorzugt steht n für 1 oder 2, besonders bevorzugt steht n für 1; das Anion ist daher besonders bevorzugt einwertig.

In Formel I steht X für das Anion einer Wasserstoffsäure mit einem pKₛ -Wert von mindestens 2, insbesondere von mindestens 3 und in einer besonderen Ausführungsform von mindestens 4 (gemessen bei 25 °C, 1 bar in Wasser oder Dimethylsulfoxid).

Der pKs -Wert der Wasserstoffsäure des Anions X beträgt vorzugsweise 2 bis 15, vorzugsweise 3 bis 15, insbesondere 3 bis 8 und besonders bevorzugt 4 bis 6.

Der pkₛ₋ Wert ist der negative dekadische Logarithmus der Säurekonstanten, K_{S}. Der pKₛ₋ Wert wird dazu bei 25 °C, 1 bar wahlweise in Wasser oder Dimethylsulfoxid als Lösemittel gemessen; erfindungsgemäß ist es daher ausreichend, wenn ein Anion entweder in Wasser oder in Dimethylsulfoxid den entsprechenden pKS- Wert hat. Dimethylsulfoxid wird insbesondere dann verwendet, wenn das Anion in Wasser nicht gut löslich ist. Zu beiden Lösemitteln finden sich Literaturangaben in Standardwerken.

Als geeignete Anionen X⁻ seien insbesondere Verbindungen mit einer oder mehreren Carboxylatgruppen (kurz Carboxylate) genannt, die den vorstehenden pkₛ Wert haben.

Als derartige Carboxylate seien insbesondere organische Verbindungen mit 1 bis 20 C-Atomen genannt, die ein oder zwei, vorzugsweise eine Carboxylatgruppe enthalten.

Es kann sich dabei sowohl um aliphatische als auch um aromatische Verbindungen handeln, wobei unter den aromatischen Verbindungen solche verstanden werden, die aromatische Gruppen enthalten. Besonders bevorzugt sind aliphatische oder aromatische Verbindungen, die außer den Sauerstoffatomen der Carboxylatgruppe keine weiteren Heteroatome enthalten oder allenfalls noch ein oder zwei Hydroxylgruppen, Carbonylgruppen oder Ethergrupen enthalten. Ganz besonders bevorzugt sind aliphatische oder aromatische Verbindungen, die außer den Sauerstoffatomen der Carboxylatgruppe keine weiteren Heteroatome enthalten.

Als Verbindungen mit zwei Carboxylatgruppen seien z.B. die Anionen der der Phthalsäure, der Isophthalsäure, der C2 bis C6 Dicarbonsäuren, z.B. Oxalsäure Malonsäure, Bernsteinsäure, Glutarsäure, Adipinsäure genannt.

Als Verbindungen mit einer Carboxylatgruppe seien die Anionen von aromatischen, aliphatischen, gesättigten oder ungesättigten C1 bis C20 Carbonsäuren, insbesondere Alkancarbonsäuren, Alkencarbonsäuren, Alkincarbonsäuren, Alkadiencarbonsäuren, Alkatriencarbonsäuren, Hydroxycarbonsäuren oder Ketocarbonsäuren oder aromatische carbonsäuren wir Benzoesäure oder Phenylessigsäure aufgeführt. Geeignete Alkancarbonsäuren, Alkencarbonsäuren und Alkadiencarbonsäuren sind auch als Fettsäuren bekannt.

Als Anionen X genannt seien insbesondere das Benzoat-Anion und die Anionen der C1 bis C20 Alkancarbonsäuren, welche gegebenenfalls durch eine oder zwei, vorzugsweise eine Hydroxygruppe substituiert sein können. Besonders bevorzugt sind das Benzoat-Anion und die Anionen der C2 bis C20 Alkancarbonsäuren; insbesondere das Acetat - Anion und Propionat-Anion; ganz besonders bevorzugt ist das Acetat-Anion.

Die Imidazoliumsalze der Formel I sind in Wasser löslich bzw. mit Wasser mischbar. Die Löslichkeit in Wasser beträgt bzw. Mischbarkeit in Wasser mindestens 50 Gramm Imidazoliumsalz, besonders bevorzugt mindestens 100 Gramm Imidazoliumsalz ganz besonders bevorzugt mindestens 200 Gramm Imidazoliumsalz und insbesondere mindestens 300 Gramm Imidazoliumsalz in 1 Liter Wasser (bei 1 bar, 21 °C).

Als Imidazoliumsalze genannt seien insbesondere
1,3 Disubstituierte Imidazoliumsalze der Formel I, wobei
R1 und R3 identisch sind
und
X für eine Verbindung mit einer Carboxylatgruppe steht,
und besonders bevorzugt
1,3 Disubstituierte Imidazoliumsalze der Formel I, wobei
R1 und R3 identisch für eine C₂ bis C₄ Alkylgruppe, insbesondere eine Ethylgruppe stehen,
R2, R4 und R5 für ein H-Atom stehen
und X für ein Acetat-Anion oder ein Propionat-Anion steht

### Zu den Ausgangsverbindungen der Herstellung

Erfindungsgemäß werden eine α -Dicarbonylverbindung, ein Aldehyd, ein Amin und die Wasserstoffsäure des Anions X⁻ miteinander umgesetzt.

Die vorstehenden Ausgangsverbindungen werden entsprechend den gewünschten Resten R1 bis R5 in Formel I gewählt.

Bei der α -Dicarbonylverbindung handelt es sich vorzugsweise um eine Verbindung der Formel II

R4-CO-CO-R5,

wobei R4 und R5 die oben genannte Bedeutung haben.

Besonders bevorzugt handelt es sich um Glyoxal.

Bei dem Aldehyd handelt es sich insbesondere um ein Aldehyd der Formel R2-CHO, wobei R2 die oben genannte Bedeutung hat. Besonders bevorzugt ist Formaldehyd; der Formaldehyd kann auch in Form von Formaldehyd freisetzenden Verbindungen wie Paraformaldehyd oder Trioxan eingesetzt werden.

Bei den Aminen handelt es sich insbesondere um primäre Amine des Typs R- NH2. Der Rest R entspricht den Resten R1 und R3 der erhaltenen Imidazoliumsalze. Wenn ein definiertes primäres Amin eingesetzt wird, erhält man ein Imidazoliumsalz, in dem die Reste R1 und R3 identisch sind. Verwendet man ein Gemisch von Aminen (z. B. Gemisch aus R'-NH2 und R"-NH2) so erhält man ein Gemisch von Imidazoiliumsalzen (Gemisch von Salzen mit R1 und R3 = R', R1 und R3 = R" und Salzen mit R1 = R' und R3 = R").
Bei der Wasserstoffsäure handelt es sich um die gewünschte Wasserstoffsäure des Anions X, vorzugsweise um eine Alkancarbonsäure, besonders bevorzugt um Essigsäure.

### Zur Durchführung des Verfahrens

Die Umsetzung der Ausgangsverbindungen erfolgt erfindungsgemäß in Wasser, einem mit Wasser mischbaren Lösemittel oder deren Gemische.

Als mit Wasser mischbare Lösemittel seien insbesondere protische Lösemittel, vorzugsweise aliphatische Alkohole oder Ether mit maximal 4 Kohlenstoffatomen, z. B. Methanol, Ethanol, Methylethylether, Tetrahydrofuran genannt. Geeignete protische Lösemittel sind mit Wasser in jedem Verhältnis mischbar (bei 1 bar, 21 °C).

Vorzugsweise erfolgt die Umsetzung in Wasser oder Gemischen von Wasser mit den vorstehenden protischen Lösemitteln. Besonders bevorzugt erfolgt die Umsetzung in Wasser.

Zur Entfernung des Wassers oder Lösemittels nach der Umsetzung werden keine hydrophoben, mit Wasser nicht mischbaren organischen Lösemittel (Kohlenwasserstoffe, z. B. Toluol) als Schleppmittel verwendet.
Das Reaktionsgemisch enthält weder während noch nach der Umsetzung hydrophobe, mit Wasser nicht mischbare organische Lösemittel, wie Kohlenwasserstoffe; insbesondere enthält das Reaktionsgemisch keine anderen Lösemittel als Wasser oder die genannten protischen Lösemittel (weder während noch nach der Umsetzung).

Die Umsetzung der Ausgangskomponenten kann bei Normaldruck und z.B. bei Temperaturen von 5 bis 100 °C, insbesondere 5 bis 50 °C, besonders bevorzugt 10 bis 40 °C erfolgen.

Die Ausgangskomponenten können in beliebiger Reihenfolge zusammengegeben werden.

Die Umsetzung kann diskontinuierlich, semi-kontinuierlich oder kontinuierlich erfolgen. Bei der semi-kontinuierlichen Fahrweise kann z. B. mindestens eine Ausgangsverbindung vorgelegt werden und die übrigen Ausgangskomponenten können zudosiert werden.

Bei der kontinuierlichen Fahrweise werden die Ausgangskomponenten kontinuierlich zusammengeführt und das Produktgemisch kontinuierlich abgeführt. Die Ausgangskomponenten können einzeln oder als Gemisch aller oder eines Teils der Ausgangskomponenten zugeführt werden. In einer besonderen Ausführungsform werden das Amin und die Säure vorab gemischt und als ein Stoffstrom zugeführt, die übrigen Komponenten können einzeln oder ebenfalls als Mischung (2. Stoffstrom) zugeführt werden.

In einer weiteren besonderen Ausführungsform werden alle Ausgangskomponenten, die Carbonylgruppen enthalten (d.h. die α -Dicarbonylverbindung, das Aldehyd und die Wasserstoffsäure des Anions X, falls es sich um ein Carboxylat handelt) vorab gemischt und gemeinsam als Stoffstrom zugeführt; das verbleibende Amin wird dann separat zugeführt.

Die kontinuierliche Herstellung kann in beliebigen Reaktionsgefäßen, z. B. einem Rührkessel durchgeführt werde. Bevorzugt wird sie in einer Rührkesselkaskade, z. B. aus 2 bis 4 Rührkersseln, oder in einem Rohrreaktor durchgeführt.

Das erhaltenen Reaktionsgemisch aufgrund von Nebenprodukten im allgemeinen dunkel gefärbt. Eine Aufhellung des Reaktionsgemisches kann im vorliegenden Fall überraschenderweise durch eine Oxidation erreicht werden.

Dazu kann das erhaltene Reaktionsgemisch mit einem Oxidationsmittel behandelt werden. Das Oxidationsmittel kann z. B. gasförmig oder flüssig sein. In Betracht kommt insbesondere gasförmiger Sauerstoff, der in geeigneter Weise, z. B. durch Druck und/oder Einleiten unterhalb der Flüssigkeitsoberfläche, mit dem Reaktionsgemisch in Kontakt gebracht wird.
In Betracht kommen insbesondere auch flüssige Oxidationsmittel, insbesondere Oxidationsmittel, welche in geeigneten, mit dem Reaktionsgemisch mischbaren Lösmitteln gelöst sind. Als Lösemittel kommt insbesondere Wasser, einem mit Wasser mischbaren Lösemittel oder deren Gemische.

Als mit Wasser mischbare Lösemittel seien insbesondere protische Lösemittel, vorzugsweise aliphatische Alkohole oder Ether mit maximal 4 Kohlenstoffatomen, z. B. Methanol, Ethanol, Methylethylether, Tetrahydrofuran genannt. Geeignete protische Lösemittel sind mit Wasser in jedem Verhältnis mischbar (bei 1 bar, 21 °C).

Vorzugsweise ist das Oxidationsmittel in Wasser oder Gemischen von Wasser mit den vorstehenden protischen Lösemitteln gelöst, besonders bevorzugt ist es in Wasser gelöst.

Geeignete Oxidationsmittel sind dem Fachmann bekannt. Oxidationsmittel sind Verbindungen mit hoher Elektronenaffinität (Elektrophilie). Stark elektrophile und damit als Oxidationsmittel geeignet Verbindungen sind z. B. Sauerstoff und Sauerstoff enthaltende Per-Verbindungen, insbesondere Wasserstoffperoxid, Metallperoxide oder organische Peroxide wie Natriumpersulfat oder tertiär Butylhydroperoxid, anorganische und organische Persäuren, z.B. Perjodsäure oder Percarbonsäuren, aber auch andere Verbindungen wie Schwefel oder Metallverbindungen hoher Wertigkeitsstufen (z.B. Eisen -III -Verbindungen, Mangandioxid, Kaliumpermanganat, Chromsäure, Chromanhydrid, Bleidioxid oder Bleitetraacetat).

Bevorzugt sind gasförmiger Sauerstoff und insbesondere Wasserstoffperoxid, vorzugsweise in Form obiger Lösungen, insbesondere als 10 bis 40 Gew. % ige Lösung.

Die Menge des Oxidationsmittels wird nach Bedarf gewählt, auf 1 Mol Imidazoliumsalz (wobei die theoretisch aus dem Reaktionsansatz erhaltenen Menge zugrunde gelegt wird) können z. B. 0,1 bis 20 Mol, vorzugsweise 0,5 bis 10 Mol Oxidationsmittel eingesetzt werden.

Die Oxidation kann z.B. bei Temperaturen von 20 bis 100 °C, insbesondere 50 bis 90 °C bei Normaldruck durchgeführt werden bis eine Aufhellung des Ansatzes eintritt. Wasser und/oder sonstiges Lösemittel (siehe oben), vorzugsweise Wasser, wird aus dem Reaktionsgemisch vorzugsweise direkt abdestilliert, d.h. es wird kein Schleppmittel, z. B. ein organisches, mit Wasser nicht mischbares Lösemittel, verwendet.

Das Wasser und/oder sonstiges Lösemittel kann vor oder nach der vorstehend beschriebenen Oxidation abgetrennt werden. Soweit eine Oxidation durchgeführt wird, erfolgt die Abtrennung des Wassers vorzugsweise danach.
Überschüssige Säure (HX) wird vorzugsweise neutralisiert, z. B. mit Alkalihydroxid, insbesondere Natriumhydroxid. Das dabei entstehende Salz (NaX) kann vorzugsweise durch Zugabe eines geeigneten Lösemittels wie Acetonitril oder Methanol ausgefällt und aus der Mischung entfernt werden. Das verwendete Lösemittel kann danach z. B. durch Destillation wieder abgetrennt werden.

Nach dem erfindungsgemäßen Verfahren werden Imidazoliumsalze durch eine nur einstufige Umsetzung in hoher Reinheit und Ausbeute erhalten. Die Maßnahmen zur Umsetzung und Aufarbeitung sind einfach durchzuführen. Unerwünschte Salze wie Ammoniumsalze, welche nur schwer entfernt werden können, werden beim erfindungsgemäßen Verfahren nicht erhalten. Überschüssige Säure kann leicht entfernt werden. Insbesondere kann das Verfahren auch kontinuierlich durchgeführt werden.

### Beispiele

### 1. Beispiel

### Herstellung von 1,3-Diethylimidazolium-Acetat (EEIM-Acetat)

### Reaktionsgleichung:

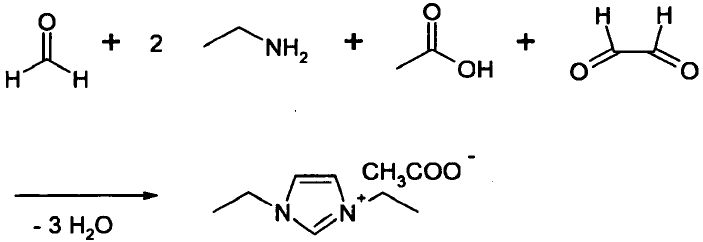

Rührgeschwindigkeiten: Reaktionsbehälter: 350 U/min

Apparatur: 6l Vierhalskolben, Teflon-Halbmondrührer, Thermometer, Kühler, Tropftrichter

### Ansatz:

| | | | | Substanz | |
|---|---|---|---|---|---|
| 252,1 | g | 8,15 | mol | Paraformaldehyd 97%ig | und |
| 325 | g | 18,04 | mol | Wasser | vorlegen |
| 1048,7 | g | 16,27 | mol | Ethylamin 70%ig in Wasser | zutropfen |
| 488,9 | g | 8,15 | mol | Eisessig | zutropfen |
| 1181,4 | g | 8,15 | mol | Glyoxal 40%ig in Wasser | zutropfen |

| Zeit | Temperatur | |
|---|---|---|
| 8:55 | 10°C | Paraformaldehyd und Wasser vorlegen, weiße Suspension, Ethylamin bei 20-30°C zutropfen, exotherme Reaktion, Eisbadkühlung |
| 9:35 | 23°C | Nach Zutropfen der Hälfte des Amins lässt die Exothermie nach, es entsteht eine klare Lösung |
| 9:50 | 23°C | Zulaufende, 30min bei RT nachrühren lassen |
| 10:35 | 25°C | Eisessig bei 20-30°C zutropfen, exotherme Reaktion, Eisbadkühlung, weißer Nebel entsteht |
| 11:10 | 25°C | Zulaufende, zwei klare Phasen, 20min bei RT nachrühren lassen |
| 11:30 | 21°C | Glyoxal bei 20-35°C zutropfen, der Ansatz wird einphasig und verfärbt sich von gelb bis schwarzbraun |
| 12:00 | 22°C | Zulaufende, über Nacht bei RT nachrühren lassen |

### Aufarbeitung:

Die erhaltene, schwarzbraune Produktmischung wurde auf 70°C erhitzen (pH 6,68) 1,5kg Wasserstoffperoxid 30%ig in ca. 1h bei 70-80°C wurden zugetropft. Nach Zulaufende war eine Aufhellung festzustellen (pH 6,33), Es wurde noch 4h bei 80°C nachgerührt (keine Gasentwicklung), es trat eine weitere Aufhellung (hellorange (pH 6,08) ein.

Die Rührgeschwindigkeit wurde auf 480 U/min erhöht und ca. 375g NaOH 40%ig zur Neutralisierung von überschüssiger Säure in ca. 1,5h zugetropft. Die Temperatur hielt sich ohne weiteres Heizen bei 65°C, der pH steigt auf 9,5 bei sehr starker Gasentwicklung (Zersetzung von Wasserstoffperoxid, H2O2). Die Temperatur stieg auf 95°C, bei pH 10,3. Der Zulauf wurde gestoppt und über Nacht bei Raumtemperatur (RT) nachgerührt (Aufhellung bis gelb).

Das Produktgemisch (pH 11,2) wurde am Rotationsverdampfer eingeengt, 1,5kg Acetonitril zugegeben und über Nacht gerührt. Das Natriumsalz der überschüssigen Säure fällt aus und wird abgetrennt. Danach wurde das Gemisch am Rotationsverdampfer eingeengt.

Insgesamt wurden 1444,14 Gramm Produkt (EEIM-acetat, 1H-NMR) erhalten.

**Tabelle 1 mit Beispielen 2 bis 11**

| | | | | |
|---|---|---|---|---|
| Abkürzungen: | | | | |
| 1 eq, 2 eq entspricht 1 mol, 2 Mol | | | | |
| MMIM: 1,3 Dimethylimidazolium | | | | |
| EEIM: 1,3 Diethylimidazolium | | | | |
| EMIM: 1-Ethyl, 3-methylimidazolium | | | | |
| | | | | |

| Beispiel Nr. | Edukt1 | Weitere Edukte | Aufarbeitung und Durchführung | Produkt (bestimmt durch (1H NMR) |
|---|---|---|---|---|
| 2 | 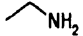 | 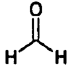 | 1Eq Ethylamin zu einer Suspension, bestehend aus 1Eq Paraformaldehyd in H2O, bei<31°C zugetropft (exotherm, Eisbad). Ca 30min nachgerührt. Mittels Eisbad auf ca 3°C abgekühlt und 1Eq Methylamin bei 3-5°C zulaufen lassen. 1Eq Eisessig bei <20°C zugetropft (exotherm, Eisbad)., 2-phasig 1Eq Glyoxal-Lös zugesetzt (exotherm, Eisbad, homogen). und bei RT 5 Tage gerührt. Ansatz bis zur Trockne am Rota eingeengt (schwarz-braunes Öl) | 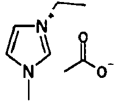 |
| | 1eq (70%ig in H2O) | 1eq (97%ig) | | |
| | 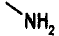 | 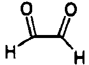 | | Gemisch von Produkten MMIM*Acetat/ EEIM*Acetat/ EMIM*Acetat 1:1.25:2.13 |
| | 1eq (40%ig in H2O) | 1eq (40%ig in H2O) | | |
| | | 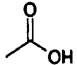 | | |
| | | 1eq pKs : 4,75 | | |
| 3 | 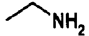 | 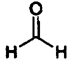 | 1Eq Ethylamin zu einer Suspension, bestehend aus 1Eq Paraformaldehyd in H2O, bei<31°C zugetropft (exotherm, Eisbad). Ca 30min nachgerührt. Mittels Eisbad auf ca 3°C abgekühlt und 1Eq Methylamin bei 3-5°C zulaufen lassen. 1Eq Glykolsäure bei <20°C zugetropft (exotherm, Eisbad), 2-phasig. | 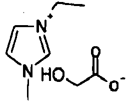 |
| | 2eq (70%ig in H2O) | 1eq (97%ig) | | |
| | | 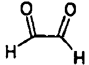 | | |
| | | 1eq (40%ig in H2O) | | |
| | | 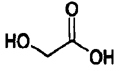 | 1Eq Glyoxal-Lös zugesetzt (exotherm, Eisbad, homogen). und bei RT 5 Tage gerührt. Ansatz bis zur Trockne am Rota | |
| | | 1eq pKs 3,0 | eingeengt (schwarz-braunes Öl) | |
| 5 | 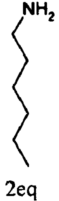 | 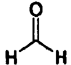 | 2eq ED1 zu einer Suspension, bestehend aus 1Eq Paraformaldehyd in H2O, bei 20-30°C zugetropft (exotherm, Eisbad). Ca 10min nachgerührt. 1Eq Propionsäure bei 20-30°C zugetropft (exotherm, Eisbad, 2-phasig, gelb-orangene trübe Suspension, ), ca 30min nachgerührt. | 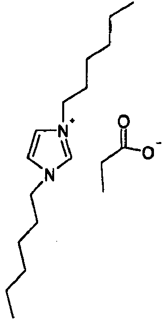 |
| | | 1eq (97%ig) | | |
| | | 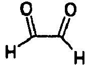 | | |
| | | 1eq (40%ig in H2O) | 1Eq Glyoxal-Lös bei 20-30°C zugetropft (exotherm, Eisbad) und bei RT ü.N. gerührt (Wasserkühlung). | |
| | | 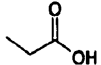 | Den heterogenen rötlichen Ansatz am Rota bei max 70°C/4mbar eingeengt, (rot-braunes Öl) | |
| | | 1eq | | |
| | | | pH (10%ige Lös in EtOH/H2O 4:1 von R1) =6.50, H2O nKF=1,2050% | |
| | | | Produkt in Acetonitril gelöst und mit 1M KOH/EtOH versetz, ca 2h bei RT gerührt, Feststoff abgesaugt, K1. Filtrat eingeengt, (Öl, enthält Feststoff) pH (10%ige Lös in EtOH/H2O 4:1 von R1) =8,40 | |
| | | | nochmals in Acetonitril über Nacht bei RT gerührt, Feststoff abgesaugt, Filtrat eingeengt, (rot braunes Öl) | |
| 6 | 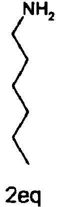 | 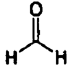 | 2eq ED1 zu einer Suspension, bestehend aus 1Eq Paraformaldehyd in H2O, bei 20-30°C zugetropft (exotherm, Eisbad). Ca 10min nachgerührt. | 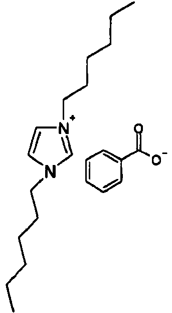 |
| | | 1eq (97%ig) | | |
| | | 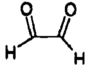 | 1Eq Benzoesäure bei 20-30°C portionsweise mit dem Spatel zugegeben (exotherm, Eisbad, gelbe klare Suspension), ca 30min nachgerührt. | |
| | | 1eq (40%ig in H2O) | 1Eq Glyoxal-Lös bei 20-30°C zugetropft (exotherm, Eisbad) und bei RT über Nacht gerührt (Wasserkühlung). | |
| | | 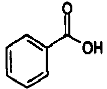 | Den heterogenen Ansatz in einen Einhalskolben überführt und am Rota bei max 70°C/4mbar eingeengt, R1 (rot-braunes Öl das durchkristallisiert). | |
| | | 1eq | Im Reaktionskolben bleibt ein gelblicher Feststoff an der Wand haften, diesen mit Toluol in einen Einhalskolben überführt und bei max 70°C/4mbar eingeengt, R2. R1 und R2 zusammen gegeben (R3) | |
| | | | R3 in EtOH/Acetonitril gelöst und mit 1M KOH/EtOH versetzt, ca 1h bei RT gerührt, Feststoff abgesaugt, K1. Filtrat eingeengt, R4 Feststoff) pH (10%ige Lös in EtOH/H2O 4:1 von R4) =8,01 | |
| | | | R4 nochmals in Acetonitril über Nacht bei Raumtemperatur (RT) T gerührt, Feststoff abgesaugt, Filtrat eingeengt, R5 (rot braunes Feststoff) | |
| 7 | 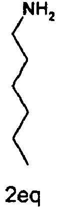 | 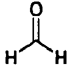 | 2eq ED1 zu einer Suspension, bestehend aus 1Eq Paraformaldehyd in H2O, bei 20-30°C zugetropft (exotherm, Eisbad). | 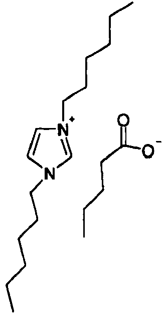 |
| | | 1eq (97%ig) | Ca 10min nachgerührt. | |
| | | 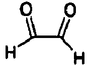 | 1Eq Propionsäure bei 20-30°C zugetropft (exotherm, Eisbad,), ca 30min nachgerührt. | |
| | | 1eq (40%lg in H2O) | 1Eq Glyoxal-Lös bei 20-30°C zugetropft (exotherm, Eisbad) und bei RT ü.N. gerührt (Wasserkühlung). | |
| | | | Den heterogenen Ansatz am Rota bei max70°C/4mbar eingeengt, R1(dunkel-braunes Öl) | |
| | | 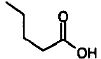 | | |
| | | 1eq | R1 in Acetonitril gelöst und mit 1M KOH/EtOH versetzt, ca 2h bei RT gerührt, Feststoff abgesaugt, K1. Filtrat eingeengt, R2 (Öl, enthält Feststoff) R2 nochmals in Acetonitril 5h bei RT gerührt, Feststoff abgesaugt, Filtrat eingeengt, R3 (rot braunes Öl) | |
| 8 | 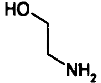 | 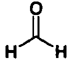 | 2Eq ED1 zu einer Lösung, bestehend aus 1Eq Formalin, 1Eq Glyoxal-Lös und 1Eq Ch3COOH bei <40°C zugetropft (exotherm, Elsbadkühlung). Ansatz rührt bei RT übers WE. Dabei wird aus der hell-braunen Lös eine dunkel-braune Lösung. | 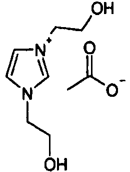 |
| | 2eq | 1eq (36.5%ig in H2O) | | |
| | | 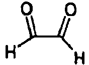 | | |
| | | 1 eq (40%ig In H2O) | Ansatz am Rota bei max 70°C/4mbar eingeengt, R1 (schwarz-braunes Öl) | |
| | | 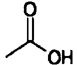 | | |
| | | 1eq | | |
| 9 | 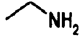 | 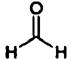 | 1Eq Glyoxal-Lös und 1Eq Formalin und 1eq Eisessig werden vorgelegt (gering exotherm) homogen. | 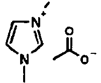 |
| | 1eq (70%igin H2O) | 1eq (36.5%ig in H2O) | | |
| | 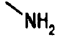 | 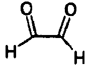 | 1Eq Ethylamin + 1eq Methylamin bei<35°C zugetropft (exotherm, Eisbadküh-lung). Die Lösung rührt bei RT über Nacht. | 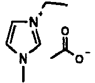 |
| | 1eq (40%ig in H2O) | | Die dunk. Braune Lös am Rota eingeengt, R1(schwarz-braunes Öl) | |
| | | 1eq (40%ig in H2O) | | 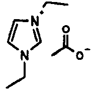 |
| | | 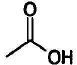 | | MMIM*Acetat/ EEIM*Acetat/ EMIM*Acetat 25:25:50 |
| | | 1eq | | |

### Beispiel 12:

kontinuierliche Herstellung von 1,3-Diethylimidazolium-Acetat (EEIM-Acetat)

Molverhältnis der Ausgangsstoffe Glyoxal (Gly), Formaldeyd (FA), Essigsäure bzw. Eisessig (ES), Ethylamin(EA):
Gly : FA : ES : EA = 1 : 1 : 1 : 2

Die Carbonylkomponenten (Gly, FA und ES) wurden vorab gemischt und als Zulauf 1 kontinuierlich zugeführt.

### Zulauf 1:

8 mol Glyoxal (40 %ig), 8 Mol Formaldehyd (40 %ig), 8 mol Eisessig,

insgesamt 2249 g

### Zulauf 2:

16 mol Ethylamin (70 %ig); 1029 g

Zulauf 1 wurde mit 8,73 ml/Stunde und Zulauf 2 mit 5,97 ml/Stunde dem ersten Rührkessel einer Rührkesselkaskade aus zwei Rührkesseln zugeführt.

Der Versuch wurde bei unterschiedlichen Temperaturen durchgeführt:

| Versuch | Verweilzeit Minuten | Temperatur °C | Ausbeute an EEIM (1H- NMR) Mol % |
|---|---|---|---|
| 12a | 22 | 24 | 85 |
| 12b | 30 | 40 | 96 |
| 12c | 30 | 60 | 79 |

## Patentansprüche

1. Verfahren zur Herstellung von 1,3 disubstituierten Imidazoliumsalzen der Formel I 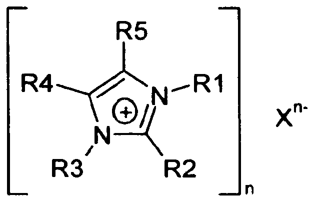 worin
R1 und R3 jeweils unabhängig voneinander für einen organischen Rest mit 1 bis 20 C-Atomen stehen
R2, R4, und R5 unabhängig voneinander für ein H-Atom oder für einen organischen Rest mit 1 bis 20 C-Atomen stehen
X für das Anion einer Wasserstoffsäure mit einem pKₛ-Wert von mindestens 2 (gemessen bei 25 °C, 1 bar in Wasser oder Dimethylsulfoxid) steht, und
n für 1, 2 oder 3 steht,
und welche eine Löslichkeit in Wasser beziehungsweise Mischbarkeit mit Wasser von mindestens 50 Gramm Imidazoliumsalz in 1 Liter Wasser (bei 1 bar, 21°C) haben,
**dadurch gekennzeichnet, dass**
a) eine α -Dicarbonylverbindung, ein Aldehyd, ein Amin und die Wasserstoffsäure des Anions X⁻ miteinander umgesetzt werden und
b) die Umsetzung in Wasser, einem mit Wasser mischbaren Lösemittel oder deren Gemische erfolgt und das Reaktionsgemisch weder während noch nach der Umsetzung Kohlenwasserstoffe enthält

2. Verfahren gemäß Anspruch 1, **dadurch gekennzeichnet, dass** R1 und R3 jeweils für den gleichen organischen Rest stehen und es sich daher um symmetrische, disubstituierte Imidazoliumsalze handelt.

3. Verfahren gemäß Anspruch 1 oder 2, **dadurch gekennzeichnet, dass** R1 und R3 unabhängig voneinander für eine C1 bis C10 Alkylgruppe stehen.

4. Verfahren gemäß einem der Ansprüche 1 bis 3, **dadurch gekennzeichnet, dass** R2, R4 und R5 unabhängig voneinander für ein H-Atom oder eine C1 bis C10 Alkylgruppe stehen

5. Verfahren gemäß einem der Ansprüche 1 bis 4, **dadurch gekennzeichnet, dass** n für 1 steht.

6. Verfahren gemäß einem der Ansprüche 1 bis 5, **dadurch gekennzeichnet, dass** der pKₛ-Wert der Wasserstoffsäure des Anions X 3 bis 8, vorzugsweise 4 bis 6 beträgt.

7. Verfahren gemäß einem der Ansprüche 1 bis 6, **dadurch gekennzeichnet, dass** X für das Anion einer Verbindung mit mindestens einer Carboxylatgruppe steht (kurz Carboxylat).

8. Verfahren gemäß einem der Ansprüche 1 bis 7, **dadurch gekennzeichnet, dass** X für das Acetat- Anion steht

9. Verfahren gemäß einem der Ansprüche 1 bis 8, **dadurch gekennzeichnet, dass** es sich bei der α -Dicarbonylverbindung um eine Verbindung der Formel II
R4-CO-CO-R5
handelt, wobei R4 und R5 die obengenannte Bedeutung haben.

10. Verfahren gemäß einem der Ansprüche 1 bis 9, **dadurch gekennzeichnet, dass** es sich bei der α -Dicarbonylverbindung um Glyoxal handelt.

11. Verfahren gemäß einem der Ansprüche 1 bis 10, **dadurch gekennzeichnet, dass** es sich bei dem Aldehyd um ein Aldehyd der Formel R2-CHO handelt, worin R2 die oben genannte Bedeutung hat.

12. Verfahren gemäß einem der Ansprüche 1 bis 11, **dadurch gekennzeichnet, dass** es sich bei dem Amin um ein Amin der Formel R1-NH2 oder ein Gemisch von Aminen mit unterschiedlichen Resten R1 handelt.

13. Verfahren gemäß einem der Ansprüche 1 bis 12, **dadurch gekennzeichnet, dass** es sich bei der Wasserstoffsäure des Anions X um eine Alkancarbonsäure handelt.

14. Verfahren gemäß einem der Ansprüche 1 bis 13, **dadurch gekennzeichnet, dass** die Umsetzung in Wasser erfolgt.

15. Verfahren gemäß einem der Ansprüche 1 bis 14, **dadurch gekennzeichnet, dass** das Wasser oder das Wasser enthaltende Lösemittelgemisch nach der Umsetzung ohne Verwendung eines Schleppmittels abdestilliert wird.

16. Verfahren gemäß einem der Ansprüche 1 bis 15, **dadurch gekennzeichnet, dass** das verfahren kontinuierlich durchgeführt wird, wobei alle Ausgangsstoffe mit Carbonyl- und Carboxylatgruppen, d.h. die α -Dicarbonylverbindung, der Aldehyd und ggb die Wasserstoffsäure des Anions X, vorab gemischt werden und dem Reaktionsgefäß gemeinsam als ein Zulauf zugeführt werden.

17. Verfahren gemäß einem Ansprüche 1 bis 16, **dadurch gekennzeichnet, dass** das erhaltene Reaktionsprodukt mit einem Oxidationsmittel behandelt wird.

## Claims

1. A process for preparing 1,3-disubstituted imidazolium salts of the formula I 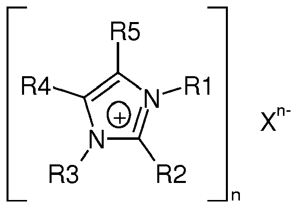 where
R1 and R3 are each, independently of one another, an organic radical having from 1 to 20 carbon atoms,
R2, R4 and R5 are each, independently of one another, an H atom or an organic radical having from 1 to 20 carbon atoms,
X is the anion of a hydrogen acid having a pKₐ of at least 2 (measured at 25°C, 1 bar in water or dimethyl sulfoxide) and
n is 1, 2 or 3,
which have solubility in water or miscibility with water a at least 50 grams of imidazolium salt per 1 liter of water (at 1 bar, 21°C),
wherein
a) an α-dicarbonyl compound, an aldehyde, an amine and the hydrogen acid of the anion X⁻ are reacted with one another and
b) the reaction is carried out in water, a solvent which is miscible with water or a mixture thereof and the reaction mixture does not comprise hydrocarbons, either during or after the reaction.

2. The process according to claim 1, wherein R1 and R3 are each the same organic radical and the imidazolium salts are therefore symmetrical, disubstituted imidazolium salts.

3. The process according to claim 1 or 2, wherein R1 and R3 are each, independently of one another, a C1-C10-alkyl group.

4. The process according to any of claims 1 to 3, wherein R2, R4 and R5 are each, independently of one another, an H atom or a C1-C10-alkyl group.

5. The process according to any of claims 1 to 4, wherein n is 1.

6. The process according to any of claims 1 to5, wherein the pKₐ of the hydrogen acid of the anion X is from 3 to 8, preferably from 4 to 6.

7. The process according to any of claims 1 to 6, wherein X is the anion of a compound having at least one carboxylate group (carboxylate for short).

8. The process according to any of claims 1 to 7, wherein X is the acetate anion.

9. The process according to any of claims 1 to 8, wherein the α-dicarbonyl compound is a compound of the formula II
R4-CO-CO-R5
where R4 and R5 are as defined above.

10. The process according to any of claims 1 to 9, wherein the α-dicarbonyl compound is glyoxal.

11. The process according to any of claims 1 to 10, wherein the aldehyde is an aldehyde of the formula R2-CHO, where R2 is as defined above.

12. The process according to any of claims 1 to 11, wherein the amine is an amine of the formula R1-NH2 or a mixture of amines having different radicals R1.

13. The process according to any of claims 1 to 12, wherein the hydrogen acid of the anion X is an alkanecarboxylic acid.

14. The process according to any of claims 1 to 13, wherein the reaction is carried out in water.

15. The process according to any of claims 1 to 14, wherein the water or the water-comprising solvent mixture is distilled off without use of an entrainer after the reaction.

16. The process according to any of claims 1 to 15 which is carried out continuously and in which all starting materials having carbonyl or carboxylate groups, i.e. the α-dicarbonyl compound, the aldehyde and, if appropriate, the hydrogen acid of the anion X, are mixed beforehand and fed together as one feed stream into the reaction vessel.

17. The process according to any of claims 1 to 16, wherein the reaction product obtained is treated with an oxidant.

## Revendications

1. Procédé de fabrication de sels d'imidazolium 1,3-disubstitués de formule I 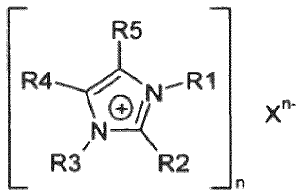 dans laquelle
R1 et R3 représentent chacun indépendamment l'un de l'autre un radical organique de 1 à 20 atomes C,
R2, R4 et R5 représentent indépendamment les uns des autres un atome H ou un radical organique de 1 à 20 atomes C,
X représente l'anion d'un hydracide d'une valeur de pKₛ d'au moins 2 (mesurée à 25 °C, 1 bar dans de l'eau ou du diméthylsulfoxyde), et
n représente 1, 2 ou 3,
et qui ont une solubilité dans l'eau ou une miscibilité avec l'eau d'au moins 50 grammes de sel d'imidazolium dans 1 litre d'eau (à 1 bar, 21 °C),
**caractérisé en ce que**
a) un composé d'α-dicarbonyle, un aldéhyde, une amine et l'hydracide de l'anion X⁻ sont mis en réaction les uns avec les autres, et
b) la réaction a lieu dans de l'eau, dans un solvant miscible avec l'eau ou leurs mélanges, et le mélange réactionnel ne contient des hydrocarbures ni pendant ni après la réaction.

2. Procédé selon la revendication 1, **caractérisé en ce que** R1 et R3 représentent chacun le même radical organique et il s'agit par conséquent de sels d'imidazolium disubstitués symétriques.

3. Procédé selon la revendication 1 ou 2, **caractérisé en ce que** R1 et R3 représentent indépendamment l'un de l'autre un groupe alkyle en C1 à C10.

4. Procédé selon l'une quelconque des revendications 1 à 3, **caractérisé en ce que** R2, R4 et R5 représentent indépendamment les uns des autres un atome H ou un groupe alkyle en C1 à C10.

5. Procédé selon l'une quelconque des revendications 1 à 4, **caractérisé en ce que** n représente 1.

6. Procédé selon l'une quelconque des revendications 1 à 5, **caractérisé en ce que** la valeur de pKₛ de l'hydracide de l'anion X est de 3 à 8, de préférence de 4 à 6.

7. Procédé selon l'une quelconque des revendications 1 à 6, **caractérisé en ce que** X représente l'anion d'un composé contenant au moins un groupe carboxylate (en abrégé carboxylate).

8. Procédé selon l'une quelconque des revendications 1 à 7, **caractérisé en ce que** X représente l'anion acétate.

9. Procédé selon l'une quelconque des revendications 1 à 8, **caractérisé en ce que** le composé d'α-dicarbonyle est un composé de formule II
R4-CO-CO-R5
dans laquelle R4 et R5 ont la signification indiquée précédemment.

10. Procédé selon l'une quelconque des revendications 1 à 9, **caractérisé en ce que** le composé d'α-dicarbonyle est le glyoxal.

11. Procédé selon l'une quelconque des revendications 1 à 10, **caractérisé en ce que** l'aldéhyde est un aldéhyde de formule R2-CHO, R2 ayant la signification indiquée précédemment.

12. Procédé selon l'une quelconque des revendications 1 à 11, **caractérisé en ce que** l'amine est une amine de formule R1-NH2 ou un mélange d'amines comprenant différents radicaux R1.

13. Procédé selon l'une quelconque des revendications 1 à 12, **caractérisé en ce que** l'hydracide de l'anion X est un acide alcanecarboxylique.

14. Procédé selon l'une quelconque des revendications 1 à 13, **caractérisé en ce que** la réaction a lieu dans de l'eau.

15. Procédé selon l'une quelconque des revendications 1 à 14, **caractérisé en ce que** l'eau ou le mélange de solvants contenant de l'eau est éliminé par distillation après la réaction sans utiliser d'agent d'entraînement.

16. Procédé selon l'une quelconque des revendications 1 à 15, **caractérisé en ce que** le procédé est réalisé en continu, toutes les matières premières contenant des groupes carbonyle et carboxylate, c.-à-d. le composé d'α-dicarbonyle, l'aldéhyde et éventuellement l'hydracide de l'anion X, étant mélangées au préalable et introduites ensemble dans le récipient de réaction sous la forme d'une alimentation.

17. Procédé selon l'une quelconque des revendications 1 à 16, **caractérisé en ce que** le produit de réaction obtenu est traité avec un oxydant.
